## Europäisches Patentamt
## European Patent Office
## Office européen des brevets

(19)

(11) Veröffentlichungsnummer: **0 149 144**
**B1**

(12) **EUROPÄISCHE PATENTSCHRIFT**

(45) Veröffentlichungstag der Patentschrift:
05.11.86

(51) Int. Cl.⁴: **C 07 C 51/573,** C 07 C 57/145, C 07 C 69/60

(21) Anmeldenummer: **84115277.0**

(22) Anmeldetag: **12.12.84**

(54) **Verfahren zur kontinuierlichen Abscheidung von Maleinsäureanhydrid aus gasförmigen Reaktionsgemischen.**

(30) Priorität: **21.12.83 DE 3346134**

(43) Veröffentlichungstag der Anmeldung:
**24.07.85 Patentblatt 85/30**

(45) Bekanntmachung des Hinweises auf die Patenterteilung:
**05.11.86 Patentblatt 86/45**

(84) Benannte Vertragsstaaten:
**BE DE FR GB IT NL**

(56) Entgegenhaltungen:
**EP-A-0 087 678**

(73) Patentinhaber: **BASF Aktiengesellschaft, Carl-Bosch-Strasse 38, D-6700 Ludwigshafen (DE)**

(72) Erfinder: **Schnabel, Rolf, Dr. Chem., Frankenstrasse 22, D-6707 Schifferstadt (DE)**
Erfinder: **Weitz, Hans-Martin, Dr. Chem., Auf dem Koeppel 40, D-6702 Bad Duerkheim (DE)**

**Beschreibung**

Diese Erfindung betrifft ein Verfahren zur kontinuierlichen Abscheidung von Maleinsäureanhydrid (MSA) aus gasförmigen Reaktionsgemischen.

Aus der DE-OS 25 43 673 ist bekannt, daß man 1,4-Butandiol aus MSA dadurch herstellen kann, daß man in einer ersten Stufe MSA mit n-Butanol verestert und den Maleinsäuredibutylester in einer zweiten Stufe katalytisch hydriert. Nach einem in der DE-PS 28 45 905 beschriebenen Verfahren führt man die 1,4-Butandiol-Bildung in einer Stufe durch, wobei man ein Gemisch aus MSA und dem Alkohol unter Verzicht auf eine vollständige Veresterung des MSA unmittelbar katalytisch hydriert.

In der DE-OS 31 06 819 wird der Vorschlag gemacht, das MSA aus einem gasförmigen Reaktionsgemisch durch eine Wäsche mit 1,4-Butandiol abzuscheiden. Das dabei erhältliche Gemisch wird zum 1,4-Butandiol hydriert. Man muß hierbei zwar kein Lösungsmittel abtrennen, doch gelingt es nicht, sowohl hohe Konzentrationen an MSA bzw. an monomeren MSA-Folgeprodukten im Absorbat zu erhalten als auch die unerwünschte Bildung von Oligomeren und Polymeren aus Butandiol und MSA zu verhindern. Beides ist aber wünschenswert, denn hohe Raum-Zeit-Ausbeuten kann man nur mit hohen Absorbat-Konzentrationen erzielen, und Oligomere und Polymere beeinträchtigen die Katalysatoren bei der nachfolgenden Hydrierung.

Versucht man, für eine Butandiol-Herstellung geeignete Gemische aus Butanol und MSA durch eine Wäsche von gasförmigem MSA enthaltenden Reaktionsgasen mit Butanol herzustellen, so ergeben sich durch den hohen Gehalt des Abgases an Butanol so erhebliche technische Schwierigkeiten, daß eine Butanolwäsche mit wirtschaftlich vertretbaren Aufwand nicht betrieben werden kann. Es bestand deshalb die Aufgabe, ein Verfahren zum Auswaschen von MSA aus Reaktionsgasen mit Lösungsmitteln zu finden, mit dem man auf möglichst wirtschaftliche Weise ein flüssiges Gemisch erhält, das sich vorteilhaft zur Gewinnung von 1,4-Butandiol hydrieren läßt.

Bei dem Verfahren der Erfindung, durch das diese Aufgabe gelöst wird, nimmt man die kontinuierliche Abscheidung von MSA aus gasförmigen Reaktionsgemischen, die man beider katalytischen Oxidation von Kohlenwasserstoffen erhält, durch Behandlung des gasförmigen Resktionsgemischs mit Lösungsmitteln so vor, daß man das MSA enthaltende gasförmige Reaktionsgemisch mit Butanol in Kontakt bringt, die dabei anfallenden gasförmigen Stoffe im Gegenstrom mit Maleinsäuredibutylester in Berührung bringt und des flüssige Verfahrensprodukt dem Sumpf entnimmt.

Nach dem erfindungsgemäßen Verfahren bringt man ein MSA enthaltendes gasförmiges Reaktionsgemisch zum Zwecke der MSA-Absorption mit Butanol in Kontakt. Als Butanol sind iso- und n-Butanol geeignet. Die Verwendung von n-Butanol als Absorptionsmittel ist bevorzugt. Die Reaktionsgaszufuhr erfolgt bei einer zweckmäßigen Ausführungsform des Verfahrens unterhalb der Oberfläche des flüssigen Butanols, z.B. durch ein Tauchrohr. Das Reaktionsgemisch kann aber auch direkt von unten in eine Absorptionskolonne eingeleitet werden, wo ihm flüssiges Butanol entgegenströmt. Die Arbeitsweise, bei der man die MSA-Absorption in einer Kolonne oder in mehreren hintereinandergeschalteten Kolonnen durchführt, ist bevorzugt. Man kann das Reaktionsgemisch auch mit gasförmigem Butanol in Kontakt bringen.

MSA enthaltende Reaktionsgemische werden z.B. nach der an sich bekannten katalytischen Oxidation von Kohlenwasserstoffen wie Butene, Butan oder auch Benzol, erhalten. Bei diesem bekannten Oxidationsverfahren strömt aus dem Reaktor ein gasförmiges Reaktionsgemisch mit einer Temperatur von 250 bis 600° C, das je Nm$^3$ bis 40 g Maleinsäureanhydrid enthält. Außerdem enthalten die gasförmigen Reaktionsgemische neben nicht umgesetztem Kohlanwasserstoff Wasser, Kohlenmonoxid und Kohlendioxid.

Als Kolonnen sind z.B. Absorptionskolonnen, wie Glockenboden-, Füllkörper- und Siebbodenkolonnen mit Bodenzahlen von 1 bis 50, vorzugsweise 5 bis 20 geeignet. Man arbeitet zweckmäßigerweise so, daß man das das MSA enthaltende gasförmige Reaktionsgemisch in den Kolonnensumpf, Butanol in den unteren Teil der Kolonne, vorteilhaft oberhalb des Kolonnensumpfes und den Meleinsäuredibutylester über den Kopf in die Kolonne einleitet. Die Temperaturen liegen im Sumpf bei 0 bis 280° C, in der Kolonne bei 0 bis 118° C und am Kopf der Kolonne bei 0 bis 100° C.

Bezogen auf 1 Mol MSA im gasförmigen Ausgangsgemisch wendet man 0,2 bis 10 Mol Butanol und 0,2 bis 20 Mol Maleinsäuredibutylester an.

Nach einer bevorzugten Arbeitsweise, bei der man die kontinuierliche Abscheidung des MSA in einer Kolonne vornimmt, werden das Gasgemisch und das Butanol wie geschildert in den Sumpf bzw. den unteren Teil der Kolonne eingeleitet. Als unterer Teil der Kolonne wird der Teil oberhalb des Sumpfes bis zum zweiten bis fünften Boden verstanden.

Oberhalb der Einleitung des Butanols, also oberhalb des zweiten bis fünften Bodens, wird eine eventuell auftretende zweite flüssige Phase, die hauptsächlich aus Wasser besteht, aus der Kolonne ausgeschleust. Aus dem mittleren Teil der Kolonne, unter dem der Bereich bis zu fünf Böden oberhalb der Entnahmestellen für die wäßrige Phase verstenden wird, schleust man ein flüssiges Gemisch aus, aus dem man in einer separaten Destillationsvorrichtung, wie einer Destillationskolonne, die leichter als Maleinsäuredibutylester (Siedepunkt 267° C) siedenden Anteile abdestilliert, die man in den unteren Teil der Kolonne zurückführt. Der bei dieser separaten Destillation als Sumpfprodukt anfellende Maleinsäuredibutylester wird der Absorptionskolonne über Kopf zugeführt. Die Kolonne wird dabei so betrieben, daß die Temperatur im untersten Boden zwischen 25 und 118° C, vorzugsweise zwischen 65 und 100° C liegt, sich im mittleren Teil der Kolonne eine Temperatur von 0 bis 65° C, vorzugsweise von 25 bis 65° C und am Kolonnenkopf eine Temperatur von 0 bis 50° C, vorzugsweise 0 bis 30° C einstellt.

Unter den Bedingungen der erfindungsgemäßen Absorption wird das MSA in der Kolonne überwiegend in den Maleinsäuremono- und -dibutylester übergeführt. Die gasförmigen Begleitstoffe des MSA werden über Kopf der Kolonne abgeleitet, während eventuell ein Teil des Wassers die Kolonne durch den Seitenabzug verläßt. Das flüssige Verfahrensprodukt, das man dem unteren Sumpf der Kolonne entnimmt, hat z.B. folgende Zusammensetzung: 0,01 bis 93 Gew.% Butanol, 0,7 bis 79 Gew.% Maleinsäuremonobutylester, 5 bis 99 Gew.% Maleinsäuredibutylester, bis zu 50 Gew.% MSA, Maleinsäure und Fumarsäure. Der Maleinsäuredibutylester enthält gewöhnlich erhebliche Anteile an dem isomeren Fumarsäurebutylester. Das flüssige Gemisch eignet sich hervorragend für die Hydrierung zur Gewinnung von 1,4-Butandiol. Hierzu empfiehlt es sich, das flüssige Gemisch zum Zwecke der Vervollständigung der Veresterung z.B. auf 100 bis 220° C zu erhitzen, wobei man das Reaktionswasser mit dem Butanol abtreibt und somit das für das erfindungsgemäße Verfahren benötigte Butanol zurückgewinnt. Der Maleinsäuredibutyl-ester kann dann auf an sich bekannte Weise zu 1,4-Butandiol hydriert werden.

Das neue Verfahren ermöglicht es, das MSA aus den Reaktionsgasen quantitativ und unter weitgehender Vermeidung von Absorptionsmittel-Verlusten in ein flüssiges Gemisch zu überführen, das eine hohe Konzentration an dem Halb- und Diester der Maleinsäure aufweist und das sich hervorragend für die Hydrierung zur Gewinnung von 1,4-Butandiol eignet. Überraschenderweise treten bei der erfindungsgemäßen Arbeitsweise störende Abscheidungen von Kristallen aus Maleinsäure und/oder Fumarsäure nicht auf.

**Beispiel 1**

(s.Figur 1)

Ein Stickstoffstrom von 200 Nl/h (Normalliter Pro Stunde) wird in einem Sättigungsgefäß mit MSA und mit Wasserdampf so beladen, daß er 1 Vol.% MSA und 5 Vol.% Wasser enthält. Der so hergestellte Gasstrom entspricht bezüglich dieser Komponenten weitgehend einem gasförmigen Reaktionsgemisch, das man bei der bekannten Luftoxidation von Butan, Butenen oder auch Benzol zum Zwecke der Herstellung von MSA erhält.

Das Gasgemisch (1) wird in einen zu Versuchsbeginn mit 1/2 l Butanol beschickten Kolben (2) bei einer Temperatur von 75° C getaucht eingeleitet. Dem Kolben wird in regelmäßigen Zeitabständen durch eine Ableitung (3) flüssiges Reaktionsgemisch entnommen, so daß der Flüssigkeitsstand im Kolben konstant bleibt.

Auf dem Kolben ist ein Schuß einer Glockenbodenkolonne (4) mit 3 Glockenböden angebracht, der mit einer Zuleitung (5) und einem Probeentnahmestutzen (6) ausgerüstet ist. Die Temperatur hält man in diesem Teil der Kolonne auf 60° C.

Über die Zuleitung (5) werden 10 ml/h n-Butanol eingeleitet. Auf dem Kolonnenteil (4) befindet sich ein mit einem Ablaßstutzen (8) versehener auf 25° C gehaltener Phasenabscheider (7), durch den man eine möglicherweise auftretende zweite flüssige Phase mit höherer Dichte (wäßrige Phase aus der Kolonne ausschleusen kann. Auf ... Phasenabscheider (7) sind weitere S...... mit insgesamt 21 Glockenböden (9) angebracht, die auf 25° C gehalten werden.

Über den Kopf der Kolonne werden 20 ml/h MSDBE zugeführt (10). Gleichzeitig verläßt ein Gasstrom den Kolonnenkopf (11).

Nach 4-tägiger Versuchsdauer stellt sich ein stationärer Zustand ein, und man erhält die folgenden Ergebnisse: Das gasförmig zugeführte MSA (10 g/h) findet sich wieder im flüssigen Gemisch, das sich im unteren Teil der Apparatur ansammelt, und zwar überwiegend in Form der Ester. Durch GC- und HPLC-Analyse des Kolbenaustrags (3) wird folgende Zusammensetzung ermittelt: 18,0 Gew.% n-Butanol, 31,0 Gew.% Maleinsäuremonobutylester, 46,4 Gew.% MSDBE und 4 Gew.% MSA, Maleinsäure und Fumarsäure. Bereits am Ablaßstutzen (6) lassen sich weder MSA, Maleinsäure, Fumarsäure noch Halbester nachweisen. Am Phasenabscheider (7) werden 4 bis 5 g/h einer wäßrigen Phase ausgeschleust, die 7 bis 8 Gew.% n-Butanol enthält. Die n-Butanol-Konzentration geht im oberen Teil der Kolonne (9) bis auf 0,4 % zurück. Mit dem Abgas (11) werden 0,42 g/h n-Butanol, 0,04 g/h MSDBE und 5 g/h Wasser ausgetragen.

**Beispiel 2**

(Vergleichsversuch)

Man verfährt wie in Beispiel 1 beschrieben, wobei man jedoch auf eine Zufuhr von Butanol verzichtet. Innerhalb eines Tages kristallisiert Maleinsäure in der Kolonne aus, so daß die Absorption abgebrochen werden muß. Erhöht man in dem Versuch die Zugabe von MSDBE auf etwa 100 ml/h, so läßt sich die störende Kristallbildung zwar verhindern, man erhält dabei jedoch ein flüssiges Verfahrensprodukt, das vor einer Hydrierung zum Zwecke der Herstellung von 1,4-Butandiol eine zusätzliche Anreicherungsatufe erforderlich machen würde.

**Beispiel 3**

(s. Figur 2)

Ein Stickstoffstrom von 200 Nl/h (Normalliter pro Stunde) wird in einem Sättigungsgefäß mit MSA und mit Wasserdampf so beladen, daß er 1 Vol.% MSA und 5 Vol.% Wasser enthält. Der so hergestellte Gasstrom entspricht einem gasförmigen Reaktionsgemisch, das man bei der bekannten Luftoxidation von Benzol, Butan oder Butenen zum Zwecke der Herstellung von MSA erhält.

Das Gasgemisch (1) wird in einen zu Versuchsbeginn mit 1/2 l Butanol beschickten Kolben (2) bei einer Temperatur von 100° C getaucht eingeleitet. Dem Kolben wird in regelmäßigen Zeitabständen durch eine Ableitung (3) flüssiges Reaktionsgemisch entnommen, so daß der Flüssigkeitsstand im Kolben konstant bleibt.

Auf dem Kolben ist ein Schuß einer Glockenbodenkolonne (4) mit 3 Glockenböden angebracht, der mit einer Zuleitung (5) und einem Probeentnahmestutzen (6) ausgerüstet ist. Die Temperatur hält man in diesem Teil der Kolonne auf 65° C.

Über die Zuleitung (5) werden 20 ml/h n-Butanol eingeleitet. Auf dem Kolonnenteil (4) befindet sich ein mit einem Ablaßstutzen (6) versehener auf 30° C gehaltener Phasenabscheider (7), durch den man eine möglicherweise auftretende zweite flüssige Phase mit höherer Dichte (wäßrige Phase) aus der Kolonne ausschleusen kann. Oberhalb des Phasenabscheiders (7), sind weitere Schüsse mit insgesamt 21 Glockenböden (9) installiert, die auf 30° C gehalten werden.

Drei Böden über dem Phasenabscheider (7) ist ein Ablaßstutzen installiert, mit dem man das flüssige Absorbat (12) aus dem darüberliegenden Kolonnenteil ausschleust und der Destillationseinrichtung (15) zuführt.

Über den Kopf der Kolonne werden 100 ml/h MSDBE zugeführt (10) und (14). Gleichzeitig verläßt ein Gasstrom den Kolonnenkopf (11).

Nach 4-tägiger Versuchsdauer stellt sich ein stationärer Zustand ein, und man erhält die folgenden Ergebnisse: Das gasförmig zugeführte MSA (10 g/h) findet sich wieder im flüssigen Gemisch, das sich im unteren Teil der Apparatur ansammelt, und zwar überwiegend in Form der Ester. Durch GC- und HPLC-Analyse des Kolbenaustrags (3) wird folgende Zusammensetzung ermittel: 25 Gew.% n-Butanol, 45 Gew.% Maleinsäuremonobutylester, 29 Gew.% MSDBE und 1 Gew.% MSA, Maleinsäure und Fumarsäure. Bereits am Ablaßstutzen (6) lassen sich weder MSA, Maleinsäure, Fumarsäure noch Halbester nachweisen. Am Phasenabscheider (7) werden 4 bis 5 g/h einer wäßrigen Phase ausgeschleust, die 4 bis g Gew.% n-Butanol enthält. Die n-Butanol-Konzentration geht im oberen Teil der Kolonne (9) bis auf weniger als 0,1 % zurück. Mit dem Abgas (11) werden 0,008 g/h n-Butanol, 0,04 g/h MSDBE und 5 bis 6 g/h Wasser ausgetragen.

Das Absorbat (12) enthält 0,8 Gew.% Wasser und 5,5 Gew.% Butanol. Es wird in eine Sambay-Destillation (15) geleitet, wo bei 20 mbar und 140° C Butanol und Wasser vom MSDBE getrennt werden. Etwa 90 g/h an MSDBE (14), der noch 0,05 Gew.% Butanol enthält, werden dem Kopf der Kolonne zugeführt, während die 16 g/h an Kopfprodukt (13), das 37 Gew.% Butanol, 5 Gew.% Wasser und 50 Gew.% MSDBE enthält, in den zweiten Boden der Absorptionskolonne geleitet werden. Der Stoffstrom (13) ist auf 65° C, der Stoffstrom (14) auf 30° C abgekühlt. Im stationären Zustand werden mit dem Stoffstrom (10) 10 ml/h MSDBE der Absorptionskolonne zugeführt.

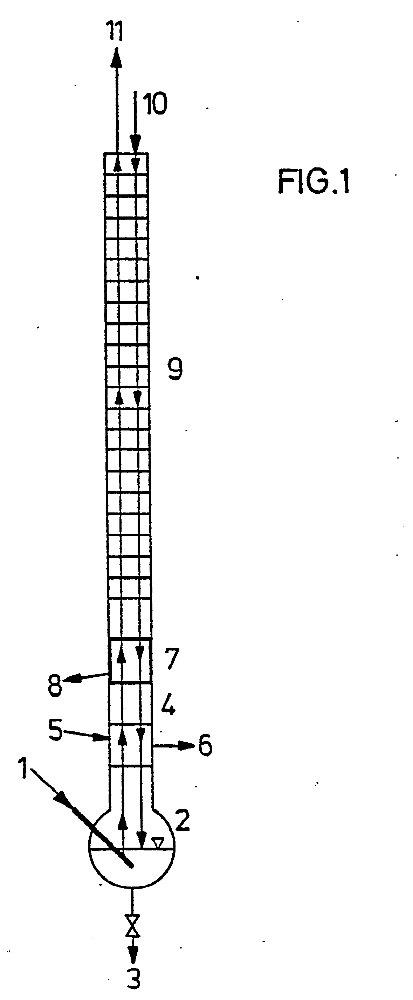

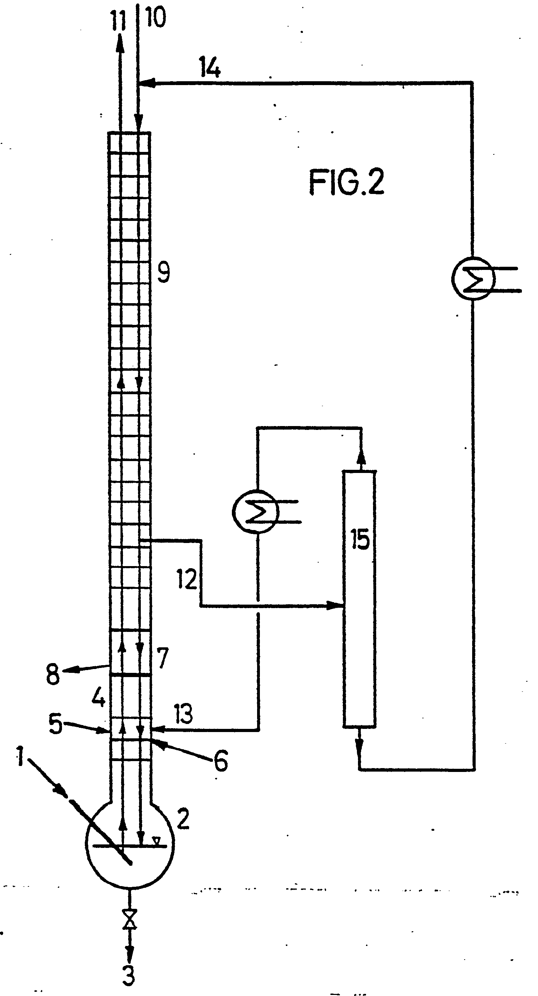

**Patentansprüche**

1. Verfahren zur kontinuierlichen Abscheidung von Maleinsäureanhydrid aus gasförmigen Reaktionsgemischen, die man bei der katalytischen Oxidation von Kohlenwasserstoffen erhält, durch Behandlung der gasförmigen Reaktionsgemische mit Lösungsmitteln, <u>dadurch gekennzeichnet</u>, daß man das das Maleinsäureanhydrid enthaltende gasförmige Reaktionsgemisch mit Butanol in Kontakt bringt, die dabei anfallemden gasförmigen Stoffe im Gegenstrom mit Maleinsäuredibutylester in Berührung bringt, und das flüssige Verfahrensprodukt dem Sumpf entnimmt.

2. Verfahren nach Anspruch 1, <u>dadurch gekennzeichnet</u>, daß man, bezogen auf 1 Mol Maleinsäureanhydrid, 0,2 bis 10 Mol Butanol und 0,2 bis 20 Mol Maleinsäuredibutylester anwendet.

3. Verfahren nach Anspruch 1, <u>dadurch gekennzeichnet</u>, daß man das das Maleinsäureanhydrid enthaltende gasförmige Reaktionsgemisch und das Butanol in den unteren Teil einer Kolonne einleitet, Wasser oberhalb dieser Einleitung aus der Kolonne ausschleust, den Maleinsäuredibutylester über den Kopf der Kolonne zuführt und das flüssige Verfahrensprodukt dem Kolonnensumpf entnimmt.

4. Verfahren nach Anspruch 1, <u>dadurch gekennzeichnet</u>, daß man das das Maleinsäureanhydrid enthaltende gasförmige Reaktionsgemisch und das Butanol in den unteren Teil einer Kolonne einleitet, aus dem mittleren Teil der Kolonne flüssiges Gemisch ausschleust, aus diesem Gemisch leichter als Maleinsäuredibutylester siedende Anteile abdestilliert und in den unteren Teil der Kolonne einleitet, und den bei dieser Destillation als Sumpfprodukt anfallenden Maleinsäurebutylester über den Kopf der Kolonne zuführt.

## Claims

1 A process for the continuous separation of maleic anhydride from a gaseous reaction mixture obtained in the catalytic oxidation of hydrocarbons, by treatment of the gaseous reaction mixture with a solvent wherein the gaseous reaction mixture containing the maleic anhydride is brought into contact with butanol, the gaseous substances thus obtained are brought into contact with dibutyl maleate flowing countercurrently thereto, and the liquid product is removed from the bottom of the column.

2. A process as claimed in claim 1, wherein from 0,2 to 10 moles of butanol and from 0,2 to 20 moles of dibutyl maleate are used per mole of maleic anhydride.

3. A process as claimed in claim 1, wherein the gaseous reaction mixture containing the maleic anhydride, and the butanol are fed into the lower section of a column, water is removed from the column above the point at which the reaction mixture and butanol are fed in, the dibutyl maleate is fed in via the top of the column, and the liquid product is removed from the bottom of the column.

4. A process as claimed in claim 1, wherein the gaseous reaction mixture containing the maleic anhydride and the butanol are fed into the lower section of a column, liquid mixture is removed from the middle section of the column, fractions which are lower boiling than dibutyl maleate are distilled off from this mixture and fed into the lower section of the column, and the butyl maleate obtained as bottoms product in this distillation is fed in via the top of the column.

## Revendications

1. Procédé pour l'extraction en continu de l'anhydride maléique de mélanges réactionnels gazeux, produits lors d'une oxydation catalysée d'hydrocarbures, par le traitement des mélanges réactionnels gazeux par des solvants, caractérisé en ce que l'on met le mélange réactionnel gazeux renfermant de l'anhydride maléique en contact avec du butanol, puis les produits gazeux présents avec du maléate de dibutyle circulant en contre-courant, le produit à séparer se retrouvant à l'état liquide dans le fond du réacteur.

2. Procédé suivant la revendication 1, caractérisé en ce que, par mole d'anhydride maléique, on met en oeuvre 0,2 à 10 moles de butanol et 0,2 à 20 moles de maléate dibutylique.

3. Procédé suivant la revendication 1, caractérisé en ce que le mélange réactionnel gazeux, renfermant de l' anhydride maléique, et le butanol sont introduits dans la partie inférieure d'une colonne, l'eau étant soutirée de la colonne en un endroit situé au-dessus du point d'introduction de ceux-ci, et le maléate de dibutyle est introduit au sommet de la colonne, tandis que le produit recherché liquide est soutiré à la base de la colonne.

4. Procédé suivant la revendication 1, caractérisé en ce que le mélange réactionnel gazeux, contenant l'anhydride maléique, et le butanol sont introduits dans la partie inférieure d'une colonne, de la partie centrale de laquelle on soutire un mélange gazeux, duquel on sépare par distillation les fractions avec un point d'ébullition inférieur à celui du maléate dibutylique, qui sont introduites dans la partie inférieure de la colonne, tandis que le maléate de dibutyle, obtenu comme résidu de la distillation, est amené au sommet de la colonne.

FIG.1

FIG.2